Europäisches Patentamt

European Patent Office

Office européen des brevets

(11) Publication number: **0 327 189**
**A2**

## (12) EUROPEAN PATENT APPLICATION

(21) Application number: 89300044.8

(22) Date of filing: 05.01.89

(51) Int. Cl.⁴: **C01B 33/28** , **B01J 29/04** ,
**C10G 35/095**

(30) Priority: 07.01.88 US 141444

(43) Date of publication of application:
**09.08.89 Bulletin 89/32**

(84) Designated Contracting States:
**BE DE FR GB IT NL SE**

(71) Applicant: **MOBIL OIL CORPORATION**
**150 East 42nd Street**
**New York New York 10017(US)**

(72) Inventor: **Chu, Cynthia Ting-Wah**
**18 Indian Run Road**
**Princeton Junction, NJ 08550(US)**
Inventor: **Han, Scott (NMN)**
**4 Valerie Lane**
**Lawrenceville, NJ 08648(US)**

(74) Representative: **Jones, Alan John**
**CARPMAELS & RANSFORD 43 Bloomsbury**
**Square**
**London, WC1A 2RA(GB)**

(54) Preparation and use of gallosilicate aromatisation catalysts.

(57) ZSM-5 catalysts containing gallium primarily present in the framework of the zeolite with little or no non-framework gallium present, are synthesised with varying reaction molar ratios of $SiO_2/Ga_2O_3$. They are useful in aromatisation, particularly of light paraffin feeds.

EP 0 327 189 A2

# PREPARATION AND USE OF GALLOSILICATE AROMATISATION CATALYSTS

The invention relates to methods of preparing a ZSM-5 aromatisation catalyst, and to the use of the catalyst in the production of high-octane aromatic products.

Zeolite catalysts have become widely-used in the processing of petroleum and in the production of various petrochemicals. Reactions such as cracking, hydrocracking, catalytic dewaxing, alkylation, dealkylation, transalkylation, isomerisation, polymerisation, addition, disproportionation and other acid-catalysed reactions may be performed with their aid.

Zeolite ZSM-5 is conventionally prepared from reaction mixtures comprising sources of silica, alumina, alkali metals, water, and a template composed of an organic nitrogen-containing cation such as tetrapropylammonium hydroxide (TPAOH). A gel or sol prepared by mixing the above ingredients is digested in a temperature range of 150°C to 200°C for about 25 to 190 hours to form a crystalline zeolite containing framework silica and alumina having a silica:alumina ratio of at least 12. The ratio can exceed 10,000. The product is then heated to remove water and activated by replacing its sodium cations with non-metallic cations, such as hydrogen and ammonium, by cation-exchange. The zeolite is defined for the purposes of the present specification by the x-ray diffraction data set forth in US-A-3,702,866, which also discloses its preparation and its use in a variety of hydrocarbon conversion processes.

It is known that the catalytic activity of zeolites may be directed to a specific hydrocarbon reaction by replacement of the framework aluminium which they conventionally contain by other metals.

According to the present inveniton ZSM-5 catalysts have gallium primarily present in the zeolite framework and contain little or no non-framework gallium. In a first method of preparing such zeolites, gallium is added to the zeolite starting mixture in the form of salts so that an $SiO_2/Ga_2O_3$ molar ratio of greater than or equal to 100/1 prevails in the reaction mixture. In a second method, gallium is added to the zeolite starting mixture in the form of salts so that an $SiO_2/Ga_2O_3$ molar ratio of less than 100/1 prevails, and sodium sulfate is added to the reaction mixture.

According to the first method a process for synthesising a crystalline gallosilicate having the structure of zeolite ZSM-5 comprises forming a reaction mixture containing sources of silica, gallia, alkali metal (M), an organic N-containing cation (R) and water and having the following composition in terms of ratios of equivalents:

$SiO_2/Ga_2O_3$ = 100-10,000
$H_2O/SiO_2$ = 5-200
$OH^-/SiO_2$ = 0-1.0
$M/SiO_2$ = 0.01-3.0
$R/SiO_2$ = 0.01-2.0

and reacting the mixture until crystals of the gallosilicate have formed. The ratio $SiO_2/Ga_2O_3$ is preferably greater than 250, advantageously greater than 500.

According to the second method the process comprises forming a reaction mixture containing sources of silica, gallia, alkali metal (M), an organic N-containing cation (R), sodium sulphate added as such and water and having the following composition in terms of ratios of equivalents:

$SiO_2/Ga_2O_3$ = 5-less than 100
$H_2O/SiO_2$ = 5-200
$OH^-/SiO_2$ = 0-1.0
$M/SiO_2$ = 0.01-3.0
$R/SiO_2$ = 0.01-2.0
$Na_2SO_4/SiO_2$ = 0.05-10

(in which M excludes Na added as $Na_2SO_4$) and reacting the mixture until crystals of the gallosilicate have formed. The value of the ratio $Na_2SO_4/SiO_2$ is preferably 0.07 to 1.0.

The preferred organic cation for synthesis of the zeolite is tetrapropylammonium. Advantageously the silica source contains less than 1% wt. Na, and may be a silica sol. After crystallisation, for which the mixture is usually reacted at 80 to 200°C for from 6 hours to 150 days, particularly at 100 to 160°C for from 25 to 190 hours, the zeolite may be dried, calcined, and converted to the hydrogen form.

The source of gallia is suitably a gallium salt, and in the reaction mixture used in both methods the following compositional relationships advantageously prevail:

$H_2O/SiO_2$ = 10-100
$OH^-/SiO_2$ = 0.02-0.2

$M/SiO_2$ = 0.02-0.7
$R/SiO_2$ = 0.05-0.5

## BRIEF DESCRIPTION OF THE DRAWINGS

Fig. 1 is a plot showing percent conversion as weight percent yield of aromatics versus the percent conversion $100-C2^{=+}$ in the presence of the [Ga]ZSM-5 catalyst of the invention.

Fig. 2 is a plot showing the aging rate of [Ga]ZSM-5 catalyst produced according to the invention in converting n-hexane to aromatic compounds. A second cycle shows improved aging over the first cycle. Aging data are also plotted for a second catalyst containing only non-framework gallium.

The starting $SiO_2/Ga_2O_3$ ratios employed in synthesis are also the ratios of material obtained in the final product. Although $SiO_2/Ga_2O_3$ ratios of greater than 10,000/1 can be used as starting ratios, zeolites of $SiO_2/Ga_2O_3$ ratio greater than 10,000/1 are of limited value in the process of the invention. The reaction mixture may be free of added alumina, although reagents such as silica sol and the gallium source will frequently contain aluminium as an impurity which will be present in the final product. The invention embraces the copresence of framework aluminium with framework gallium, and even the deliberate addition of a source of alumina to the reaction mixture in the case of <100:1 $SiO_2/Ga_2O_3$ syntheses.

The reaction mixture is heated to a temperature of from 100 to 175° C for a period of about six hours to sixty days. A preferred temperature range is from about 150 to 175° C for twelve hours to eight days. The reaction is advantageously conducted in an autoclave, optionally with agitation, until crystals form. The solid product is separated form the reaction medium by cooling the mixture to room temperature and then washing.

The product is dried at elevated temperatures, for example at 110° C for eight to twenty-four hours or at room temperature under vacuum. Its organic content may be removed by calcination, suitably in air or oxygen at 538° C for four hours. The zeolite is preferably ion exchanged, for example with $NH_4NO_3$, and then activated by calcination at 538° C to convert it to the hydrogen form.

Suitable sources of gallium include gallium sulfate, gallium nitrate, and gallium chloride. Suitable silica sources include silica hydrosol, silica gel, and silicic acid. The template may be any of the organic templates used to synthesise ZSM-5, for example tetrapropylammonium hydroxide, tetrapropylammonium bromide or tetrapropylammonium chloride or other organic compounds known to those skilled in the art as successfully-used templates. The order of adding or mixing the ingredients is not critical and the reaction mixture can be prepared batchwise or continuously.

A quantitative amount of sodium sulfate added to a zeolite reaction mixture of $SiO_2/Ga_2O_3$ molar ratios less than 100 improves the crystallinity of the product, and thus the amount of framework gallium in the product. When the $SiO_2/Ga_2O_3$ ratio of the zeolite reaction mixture is ≥ 100 products of 100% crystallinity or thereabouts are obtained in the absence of sodium sulfate.

The [Ga]ZSM-5 zeolites produced according to the invention can have a wide variety of cations associated therewith, just as in the case of aluminosilicate forms of the zeolite which can be replaced by a wide variety of other cations according to techniques well known in the art. Typical replacing cations include hydrogen, ammonium and metal cations, including mixtures of the same. Of the replacing metallic cations, particular reference is made to cations of such metals as manganese and calcium, as well as metals of Group II of the Periodic Table, e.g., zinc, and Group VIII of the Periodic Table, e.g., nickel.

Catalysts comprising zeolites prepared according to the invention may be formed in a wide variety of particle sizes. Generally speaking, the particles can be in the form a powder, a granule, or a molded powder, such as an extrudate having a particle size sufficient to pass through a 2-mesh (Tyler) screen and retained on a 400-mesh (Tyler)screen. In cases where the catalyst is molded, such as by extrusion, the product can be extruded before drying or partially dried and then extruded. It may be desirable to composite the zeolite with other materials resistant to temperatures and conditions employed in organic conversion processes. Such materials include active and inactive materials, synthetic or naturally-occurring, as well as inorganic materials such as clays, silica, and/or metal oxides such as alumina. The use of an active material in combination with a ZSM-5 catalyst can improve the conversion and/or selectivity of the catalysts in certain organic conversion processes. Inactive materials combined with the catalyst serve as diluents to control the amount of conversion in a given process so that products can be obtained economically and in an orderly manner without employing other means for controlling the rate of reaction. Suitable binder/matrix materials for compositing with zeolites are fully discussed in our EP-A-1695.

According to a further aspect of the invention a process for converting an aliphatic feed comprising paraffins, olefins and/or naphthenes of 2 to 12 carbon atoms to a product comprising at least 30% wt. aromatics comprises contacting said feed at 400-650°C, 1-15 bar and 0.1-10 WHSV with a catalyst comprising a gallosilicate zeolite ZSM-5. The feed preferably contacts the catalyst at 450 to 550°C, 1.35-5 bar and -0.5-2.5 LHSV, and the catalyst is advantageously a composite of the gallosilicate with a silica, alumina and/or clay binder, although other compositing materials may be used. In continous processes the catalyst is periodically subjected to regeneration, which may be in a hydrogen atmosphere for at least part of the regeneration procedure.

The catalyst of this inveniton can thus be used for the production of high-octane gasoline products. The catalyst shows high activity and selectivity and improved stability for reactions such as cracking and light paraffin upgrading, notably light paraffin upgrading. More particularly, the catalyst can be used for converting $C_2$ to $C_{12}$ paraffins, olefins and naphthenes to high-octane aromatics.

The reaction mixtures employed in the illustrative Examples which follow manifest the following compositional ratios: $H_2O/SiO_2 = 40$; $OH^-/SiO_2 = 0.05$; $Na^+/SiO_2 = 0.05$; and $TPA^+/SiO_2 = 0.1$.

$SiO_2/Ga_2O_3$ is varied from Example to Example.

## EXAMPLE 1

The $SiO_2/Ga_2O_3$ starting ratio was 100/1. 48.0 grams of silica sol (30.0% silica) were mixed with a solution containing 1.03 grams of $Ga_2(SO_4)_3$ and 0.48 grams of NaOH in 139 grams of water. 6.38 grams of tetrapropylammonium bromide was added to the mixture and the pH was adjusted to pH 12-12.5 by the addition of small amounts of sodium hydroxide. The mixture was placed in an autoclave and heated at 145-150°C and autogenous pressure for two to three days. The product was established as being 100 percent crystalling ZSM-5. Ga-NMR reveals that the product contains gallium which is present primarily in the framework of the zeolite and that the zeolite contains little or no non-framework gallium.

## EXAMPLES 2-5

These Examples follow the procedure of Example 1, except that their starting $SiO_2/Ga_2O_3$ molar ratios were 200/1, 250/1, 350/1 and 500/1 respectively. The starting amounts of $Ga_2O_3$ are listed in Table I.

The crystallinity of all four products was determined to be 100%. Ga-NMR reveals that they contain gallium which is present primarily in the framework of the zeolite and that the zeolites contain little or no non-framework gallium.

## EXAMPLE 6

This Example follows the procedures of Example 1 but differs therefrom by the use of a starting $SiO_2/Ga_2O_3$ molar ratio of <100 and by the presence of an amount of sodium sulfate appropriate to give a desired product. A mixture containing 27.8 grams of silica gel (30.0% silica) in 40 grams of water and 6.0 grams of NaOH was mixed with 2.2 grams of $Ga_2(SO_4)_3$ to yield a $SiO_2/Ga_2O_3$ hydrogel of mole ratio 90. 46 grams of $H_2O$ and 10.6 grams of $H_2SO_4$ were added together with 12.3 grams of tetrapropylammonium bromide and 9.7 grams of sodium sulfate. The pH of the mixture was adjusted to 10 with sodium hydroxide. The mixture was placed in an autoclave and heated at 160°C and autogenous pressure for seven days.

Ga-NMR reveals that the product contains gallium which is primarily present in the framework of the zeolite and that the zeolite contains little or no non-framework gallium.

## EXAMPLE 7

This Example effectively repeats Example 6 except that sodium sulfate was not added to the reaction mixture. A mixture containing 27.8 grams of silica gel (30.0% silica) in 86 grams of water was mixed with 2.2 grams of $Ga_2(SO_4)_3$ in 84 grams of $H_2O$ to yield a $SiO_2/Ga_2O_3$ hydrogel of molar ratio 90. 10.6 grams of $H_2SO_4$ and 12.3 grams of tetrapropylammonium bromide were added and the resulting mixture adjusted to pH 10 with sodium hydroxide solution. The mixture was placed in an autoclave and heated at 160° C and autogenous pressure for seven days.

Ga-NMR reveals that 47% of the gallium content of this product is non-framework gallium.

Nuclear Magnetic Resonance (NMR) is a proven effective tool for identifying, and quantitatively determining, the presence of gallium in the framework of zeolite materials. The identification of the presence of framework gallium in such materials using NMR is called Ga-NMR. The use of Ga-NMR to determine framework gallium is described in the Journal of Molecular Catalysis, 1985, 31, 355. Whether or not a [Ga]-ZSM-5 product contains gallium primarily present in the framework of the zeolite is determined by measuring gallium content by Ga-NMR and comparing this measurement with the overall gallium content for a given sample as determined by elemental analysis.

Table I sets forth the results showing that the [Ga]ZSM-5 synthesised in Examples 1-6 contain gallium which is primarily present in the framework and contain little or no non-framework gallium. The result is contrasted with the result obtained by the method of Example 7, the product of which contains both framework and significant amounts of non-framework gallium.

The catalysts of Examples 1 to 6 show high activity and stability compared with catalysts which contain non-framework gallium in reactions such as light paraffins upgrading: $C_2$-$C_{12}$ paraffins, olefins and naphthenes can be readily converted to aromatic compounds having high octane ratings such as benzene, toluene, xylenes and mixtures thereof.

TABLE I

| Ga-MASNMR and Analytical Data for Determining Presence of Framework Gallium in [Ga]ZSM-5 Samples . | | | | | | |
|---|---|---|---|---|---|---|
| | Starting Grams $Ga_2(SO_4)_3$ | Starting Molar Ratio $SiO_2/Ga_2O_3$ | % Ga, NMR | % Ga, Anal. | % Non-Framework Ga | % $Al_2O_3$ Anal. |
| Ex.1 | 1.03 | 100/1 | 2.3 | 2.04 | 0 | 0.058 |
| Ex.2 | 0.51 | 200/1 | 1.6 | 1.34 | 0 | . 0.065 |
| Ex.3 | 0.41 | 250/1 | 0.88 | 0.79 | 0 | 0.666 |
| Ex.4 | 0.29 | 350/1 | 0.67 | 0.58 | 0 | 0.064 |
| Ex.5 | 0.21 | 500/1 | 0.46 | 0.43 | 0 | 0.068 |
| $SiO_2/Ga_2O_3$ ratio < 100 with the addition of sodium sulfate. | | | | | | |
| Ex.6 | 2.2 | 90/1 | 1.6/1.0* Avg. = 1.3 | 1.16 | Avg. = 0 | 0.037 |
| Avg. = 0 | | | | | | |
| Ex.7 | 2.2 | 90/1 | 0.81 | 1.54 | .47 | 0.010 |

* Two NMR determinations.

## EXAMPLE 8

N-hexane and the [Ga]ZSM-5 prepared in accordance with Example 6 were charged into a reactor which was adjusted at 538° C at atmospheric pressure at a liquid hourly space velocity of 0.59. The activity of the catalyst is measured by percent conversion, defined as $100-C2^{=+}$ aromatisables: $C2^{=+}$ aromatisables are defined as all non-aromatic hydrocarbons except methane and ethane. In a first run, the reaction yielded thirty-five percent by weight or aromatics and twenty-seven percent weight of $C_2$ to $C_2$ paraffins with a sixty-two pecent conversion.

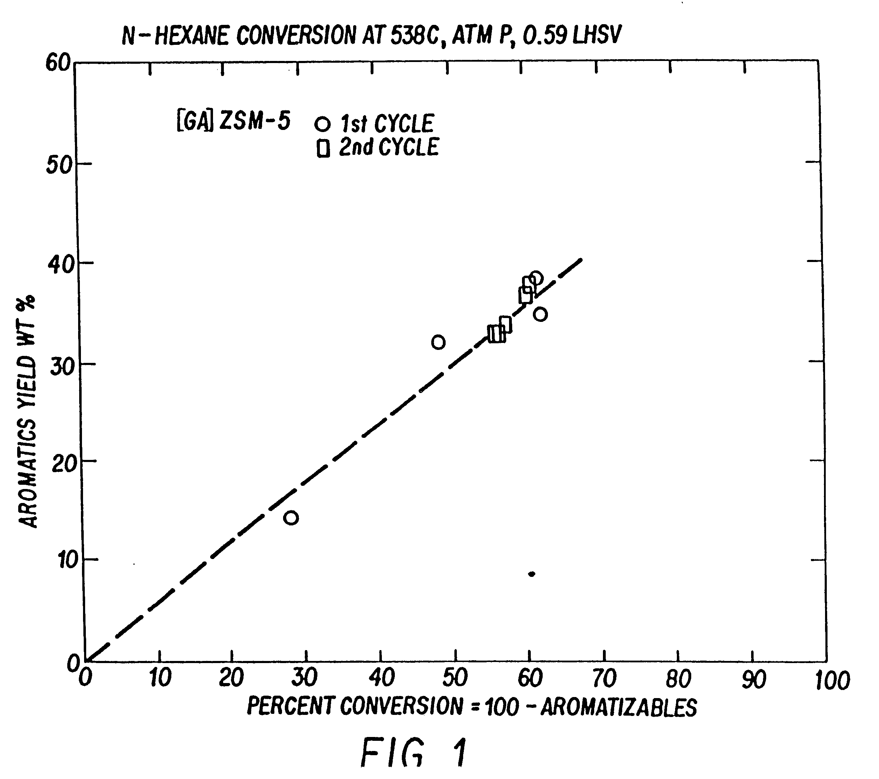

Fig. 1 is a plot showing the weight percent yield of aromatics at various conversion percentages for starting material hexane, identified as an aromatisable, using the catalyst of Example 6. The plotted points of Fig. 1 are in Table II below:

TABLE II

| CONVERSION DATA | | |
|---|---|---|
| 1st Cycle | Aromatics Yield Wt % | 100-C2$^{=+}$ |
| 1 | 35 | 62 |
| 2 | 38 | 62 |
| 3 | 33 | 48 |
| 4 | 14 | 28 |
| 2nd Cycle | | |
| 1 | 37 | 61 |
| 2 | 38 | 60 |
| 3 | 33 | 58 |
| 4 | 32 | 56 |

In the first cycle above, the catalyst converts hexane to aromatic products and aromatic intermediates which subsequently produce aromatics. As is seem from the plot, conversion decreases as the catalyst remains on line. The catalyst was regenerated and was used in Cycle 2.

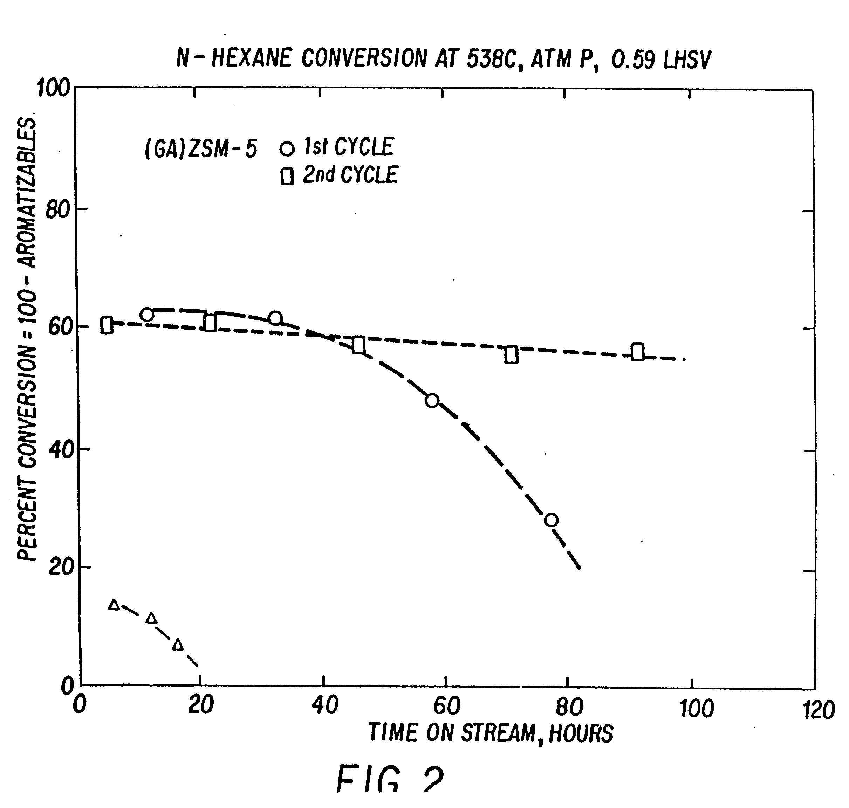

Catalysts produced in accordance with the invention have been found to have an improved aging rate. Fig. 2 is an aging plot of conversion of n-hexane to aromatic compounds versus time on stream. The plotted points of Fig. 2 are reproduced in Table III below. The catalyst was used for 90 hours in a first cycle and its activity, i.e., the conversion of n-hexane to products, rapidly decreased after forty hours of use. However, after the regeneration and the use of this catalyst in a hydrogen atmosphere, the catalyst showed sustained and constant activity for over 100 hours in a second cycle as shown in Fig. 2 and in Table III below.

Fig. 2 also plots the aging data for a second catalyst which contains gallium only as non-framework gallium. Such a catalyst, as shown by the plotted points represented as triangles, shows a very poor aging rate, and after only 20 hours on line has aged markedly.

TABLE III

| AGING DATA | | |
|---|---|---|
| 1st Cycle | % Conversion 100-C2$^{=+}$ | Time on Stream Hours |
| 2 | 62 | 12 |
| | 62 | 35 |
| | 48 | 60 |
| | 28 | 78 |
| 2nd Cycle | | |
| | 61 | 22 |
| | 60 | 48 |
| | 58 | 75 |
| | 56 | 95 |

Initial regeneration has been accomplished by contacting the catalyst with elemental $H_2$ at temperatures between 800 and 1000° F (427 to 538° C). Hydrogen regeneration usually removes soft deposits of coke but does not always completely restore the original level of catalyst activity, and oxygen regeneration is used to burn hard coke residue off the catalyst. Suitable regeneration techniques are described in US-A-4,276,437.

**Claims**

1. A process for synthesising a crystalline gallosilicate having the structure of zeolite ZSM-5 with comprises forming a reaction mixture containing sources of silica, gallia, alkali metal (M), an organic N-containing cation (R) and water and having the following composition in terms of ratios of equivalents:

$SiO_2/Ga_2O_3$ = 100-10,000
$H_2O/SiO_2$ = 5-200
$OH^-/SiO_2$ = 0-1.0
$M/SiO_2$ = 0.01-3.0
$R/SiO_2$ = 0.01-2.0

and reacting the mixutre until crystals of the gallosilicate have formed.

2. A process according to claim 1 wherein the ratio $SiO_2/Ga_2O_3$ is greater than 250.

3. A process according to claim 1 or claim 2 wherein the ratio $SiO_2/Ga_2O_3$ is greater than 500.

4. A process for synthesising a crystalline gallosilicate having the structure of zeolite ZSM-5 which comprises forming a reaction mixture containing sources of silica, gallia, alkali metal (M), an organic N-containing cation (R), sodium sulphate added as such and water and having the following composition in terms of ratios of equivalents:

$SiO_2/Ga_2O_3$ = 5-less than 100
$H_2O/SiO_2$ = 5-200
$OH^-/SiO_2$ = 0-1.0
$M/SiO_2$ = 0.01-3.0
$R/SiO_2$ = 0.01-2.0
$Na_2SO_4/SiO_2$ = 0.05-10

(in which M excludes Na added as $Na_2SO_4$) and reacting the mixture until crystals of the gallosilicate have formed.

5. A process according to claim 4 wherein the value of the ratio $Na_2SO_4/SiO_2$ is 0.07 to 1.0.

6. A process according to any preceding claim wherein R is tetrapropylammonium.

7. A process according to any preceding claim wherein the silica source contains less than 1% wt. Na.

8. A process according to any preceding claim wherein the source of silica is a silica sol.

9. A process according to any preceding claim wherein said mixture is reacted at 80 to 200°C for from 6 hours to 150 days.

10. A process according to claim 9 wherein the mixture is reacted at 100 to 160°C for from 25 to 190 hours.

11. A process according to any preceding claim wherein the source of gallia is a gallium salt.

12. A process according to any preceding claim wherein the reaction mixture:

$H_2O/SiO_2$ = 10-100
$OH^-/SiO_2$ = 0.02-0.2
$M/SiO_2$ = 0.02-0.7
$R/SiO_2$ = 0.05-0.5

13. A process according to any preceding claim wherein the reaction mixture contains no aluminium added otherwise than as an impurity.

14. A process according to any preceding claim wherein the product gallosilicate is dried and calcined.

15. A process according to any preceding claim wherein the product gallosilicate is converted by base exchange into the hydrogen form.

16. A process for converting an aliphatic feed comprising paraffins, olefins and/or naphthenes of 2 to 12 carbon atoms to a product comprising at least 30% wt. aromatics which comprises contacting said feed at

temperature 400-650°C
pressure 1-15 bar
WHSV 0.1-10

with a catalyst comprising a gallosilicate zeolite ZSM-5.

17. A process according to claim 16 wherein the feed contacts the catalyst at 450 to 550°C, 1.35-5 bar and 0.5-2.5 LHSV.

18. A process according to claim 16 or 17 wherein the catalyst is a composite of the gallosilicate with a silica, alumina and/or clay binder.

19. A process according to any of claims 16 to 18 wherein the catalyst is periodically subjected to regeneration in a hydrogen atmosphere.

20. A process according to any of claims 16 to 19 wherein the gallosilicate contains no non-framework NMR-detectable gallium.

N-HEXANE CONVERSION AT 538C, ATM P, 0.59 LHSV

[GA] ZSM-5    ○ 1st CYCLE
              □ 2nd CYCLE

AROMATICS YIELD WT %

PERCENT CONVERSION = 100 - AROMATIZABLES

FIG 1

EP 0 327 189 A2

N-HEXANE CONVERSION AT 538C, ATM P, 0.59 LHSV

(GA)ZSM-5    ○ 1st CYCLE
             □ 2nd CYCLE

PERCENT CONVERSION = 100 - AROMATIZABLES

TIME ON STREAM, HOURS

FIG 2

EP 0 327 189 A2